# EUROPEAN PATENT APPLICATION

(11) **EP 0 800 872 A2**
(43) Date of publication of application: **15.10.1997**
(21) Application number: 97105835.9
(22) Date of filing: 09.04.1997
(51) Int. Cl.: B09B 3/00

(54) **Method for treating municipal solid waste and the like, and plant for carrying out the method**

(30) Priority: 11.04.1996 IT MI960698
(71) Applicant: Castelli, Elio, I-24024 Gandino (Bergamo) (IT); Agosti, Artemisio, I-24100 Bergamo (IT)
(72) Inventor: Castelli, Elio, I-24024 Gandino (Bergamo) (IT); Agosti, Artemisio, I-24100 Bergamo (IT)
(74) Representative: Modiano, Guido, Dr.-Ing.

(57) **Abstract**

A method for treating municipal solid waste and the like comprising in the steps of: triturating (2) the waste arriving from a storage area (1) and draining the percolate; homogenizing (4) the triturated waste, with further percolate draining; introducing the homogenized waste into a compaction and pressing chamber (6); applying pressure to reduce the volume and bring the density to 1.4-1.5 tons/m³ with final percolate draining; introducing the compacted block in a pasteurization container (20); subjecting the container to thermal pasteurization (40) without combustion to stabilize the organic materials and to destroy the microorganisms which produce fermentation; and subjecting the pasteurized block to cooling (50) with subsequent extraction from the container.

## Description

The present invention relates to a method for treating municipal solid waste and the like, and to the plant for carrying out the method.

It is known that one of the currently strongly felt problems relates to the difficulties encountered in the disposal of municipal solid waste and the like, since the methods conventionally used generally cause pollution and are poorly accepted by the populations where the plants are installed.

In the most widely used system, the municipal solid waste is buried in landfills constituted by basins which are rendered impermeable by means of a bottom geomembrane, above which the municipal solid waste is placed in layers alternated with soil.

Because of its intrinsic characteristics, municipal solid waste requires the use of large storage basins, drainage structures, and the subsequent recovery of the biogas produced during the fermentation phase which is typical of all organic material in anaerobic conditions.

Currently used landfills entail drawbacks for the environment, since light materials are dispersed into the environment due to wind action, spreading out unpleasant odors caused by the fermentation of the municipal solid waste, which is not always covered daily.

There is also a great proliferation of insects and rodents and of birds such as gulls which, by feeding off the waste, spread it into the surrounding environment.

Landfills also entail severe fire problems; by burning the waste at low temperature, said fires can generate noxious substances.

In addition to the above-cited drawbacks, it is necessary to "farm" the landfill even for many years after closing it, continuing to extract and burn the biogas and repairing the subsidences and settlements caused by the destruction of organic material through biogas production.

Other methods which have already been studied provide for the heat-assisted compaction and pasteurization of the waste, with the possibility of inactivating the organic substances contained in the municipal solid waste and the like, avoiding fermentation and the formation of biogas.

These methods, which have proved to be undoubtedly valid from a conceptual point of view, however currently have the drawback that they require subsequent steps for the compaction of the waste which are considerably expensive and the use of waste compaction containers which have a very large mass in order to withstand the final compaction pressures, with the consequent need for a considerable expenditure of heat energy for the thermal pasteurization of said waste, since it is necessary to also bring the container to the required temperature.

A principal aim of the present invention is to solve the above-described problems, by providing a method for treating municipal solid waste and the like which allows to drastically reduce the volume of municipal solid waste and the like while using containers having a reduced mass, so that the addition of heat energy during final pasteurization is considerably reduced.

Within the scope of this aim, a particular object of the present invention is to provide a method in which it is possible to provide gradual compaction in a single step and to also have compacted waste containment containers which are much easier to handle.

Another object of the present invention is to provide a method which allows to radically eliminate the environmental problems linked to the fermentation of organic waste, with all negative side effects related thereto.

Another object of the present invention is to provide a plant for carrying out the method for treating municipal solid waste and the like which is structurally simple and management whereof is assuredly advantageous from a purely economical point of view.

This aim, these objects, and others which will become apparent hereinafter are achieved by a method for treating municipal solid waste and the like, according to the present invention, characterized in that it consists in triturating the waste arriving from a storage area and draining the percolate; in homogenizing the triturated waste, with further percolate draining; in introducing the homogenized waste into a compaction and pressing chamber; in applying pressure to reduce the volume and bring the density to 1.4-1.5 tons/m³ with final percolate draining; in introducing the compacted block in a pasteurization container; in subjecting the container to thermal pasteurization without combustion to stabilize the organic materials and to destroy the microorganisms which produce fermentation; and in subjecting the pasteurized block to cooling with subsequent extraction from the container.

Further characteristics and advantages of the present invention will become apparent from the following detailed description of some preferred but not exclusive embodiments of a plant for treating municipal solid waste and the like, illustrated only by way of non-limitative example in the accompanying drawings, wherein:
figure 1 is a block diagram of the method for treating municipal solid waste according to the present invention;
figure 2 is a schematic view of the plant, illustrating the compaction chamber;
figure 3 is a schematic transverse sectional view of the compaction chamber;
figure 4 is a longitudinal sectional view of the step for compacting the triturated waste;
figure 5 is a view of the step for transferring the compacted waste into the pasteurization container;
figure 6 is a view of the cover for closing the container;
figures 7 and 8 are schematic views of a different embodiment of the plant;
figures 9 and 10 set forth schematically a plant with a movable closure panel in the compaction chamber.

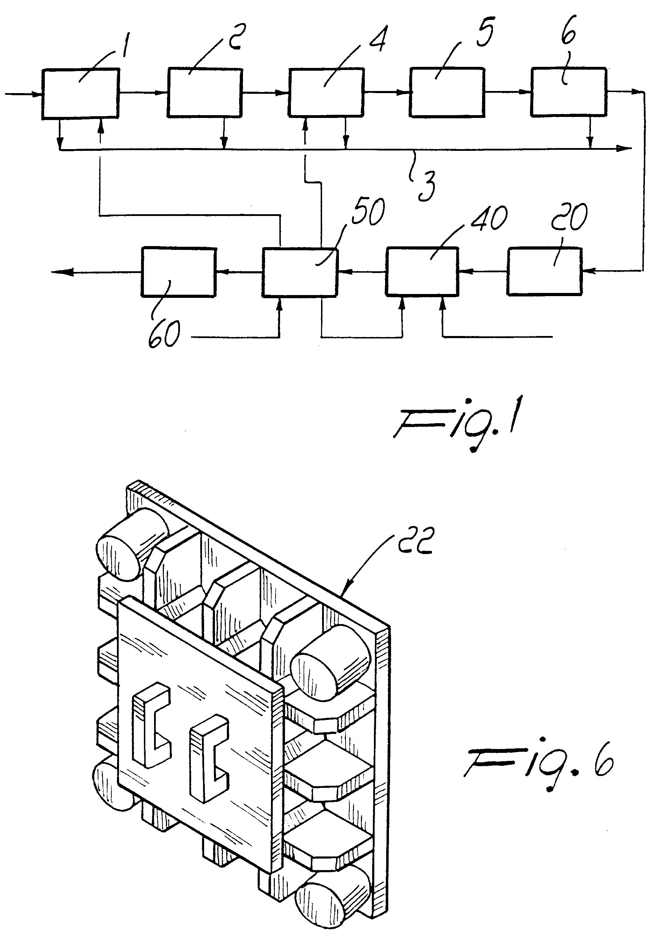

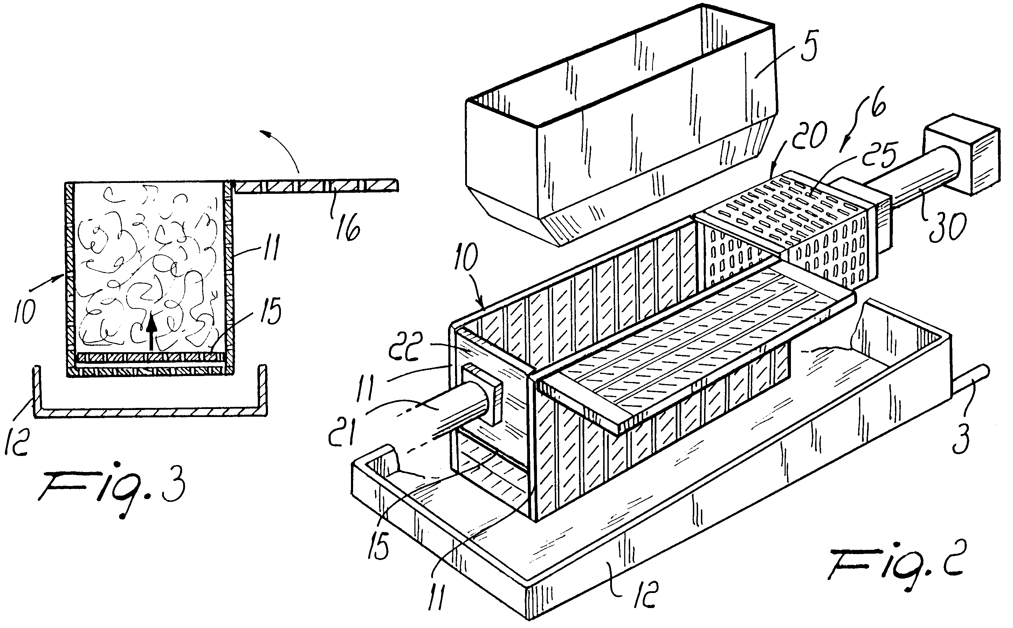

With reference to the above figures, the municipal solid waste and similar solid waste arrives at the plant in an irregular fashion and is introduced in a storage area designated by the reference numeral 1. The waste, which initially has a very low density which can be estimated at approximately 0.3 tons/m³, are triturated in the triturator 2; this trituration has the purpose of reducing the larger pieces, mixing the various materials, releasing part of the liquid substances which are present, and draining the percolate, which is fed into a storage tank by means of a discharge duct designated by the reference numeral 3.

The triturated material is fed into a homogenizer 4 provided with a homogenizing tank which in practice acts as a buffer storage unit for the subsequent steps.

Hot air produced from the cooling of the pasteurized block, as better described hereinafter, can be injected into said tank so as to evaporate the contained liquids.

The homogenized material is fed, by means of belts or similar elements, to a weighing station 5 which in practice has a hopper which is installed on load cells for feeding into a compaction unit, generally designated by the reference numeral 6.

Said compaction unit, as will become apparent hereinafter, has a compact ion and pressing chamber, generally designated by the reference numeral 10, having the purpose of drastically reducing the volume, bringing the waste to a density of approximately 1.4-1.5 tons/m³.

The chamber 10 has side walls 11 which can be provided by means of steel strips spaced from each other by approximately 3 mm, arranged side by side along a longitudinal or transverse direction, and allowing the escape of the percolation liquid of the solid waste, or having holes or slots for the escape of the liquid, the chamber being located above a collecting tank 12 which, through the duct 3, then sends the percolate produced by final draining towards the storage tank.

Inside the side walls 11 there is provided a movable bottom 15 which is also provided with holes and is in the lowered position in the initial step; once waste has been introduced, said bottom is made to rise, so as to produce, for the chamber, which is closed at the top by a cover 16, a transverse cross-section corresponding to the transverse cross-section of a pasteurization container 20, which is aligned with the chamber on the opposite side with respect to a compaction piston 21, whereto a cover 22 for closing the container 20 is connected.

The cover 22 is provided with engagement means in order to couple to the piston 21, which is actuated with a logarithmically-increasing pressure, so as to allow the cycle for reducing the compaction volume of the material in the chamber to occur continuously until a pressure of approximately 3500 N/cm² on the material is achieved. The cover is also provided with a system for automatic coupling to the pasteurization container once the desired pressure has been reached and the compacted block has been transferred into the container 20.

A contrast piston 30 is provided opposite to the compaction piston 20; in the initial step, said piston is inserted through the container so as to move a bottom closure panel 31, structurally similar to the cover 22, at the end of the chamber 10, so that the step during which the high compaction pressure is applied occurs entirely inside the chamber 10. The bottom closure panel 31 is provided with engagement means so that it can couple to the contrast piston 30 and with a system for automatically engaging the pasteurization container 20, once the compacted block has been transferred into the container 20.

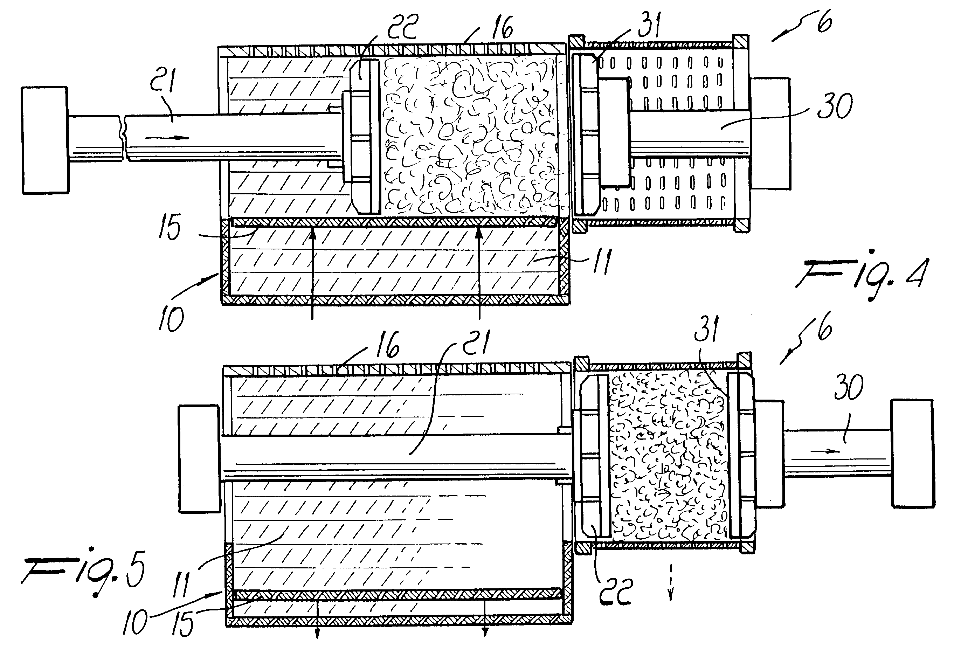

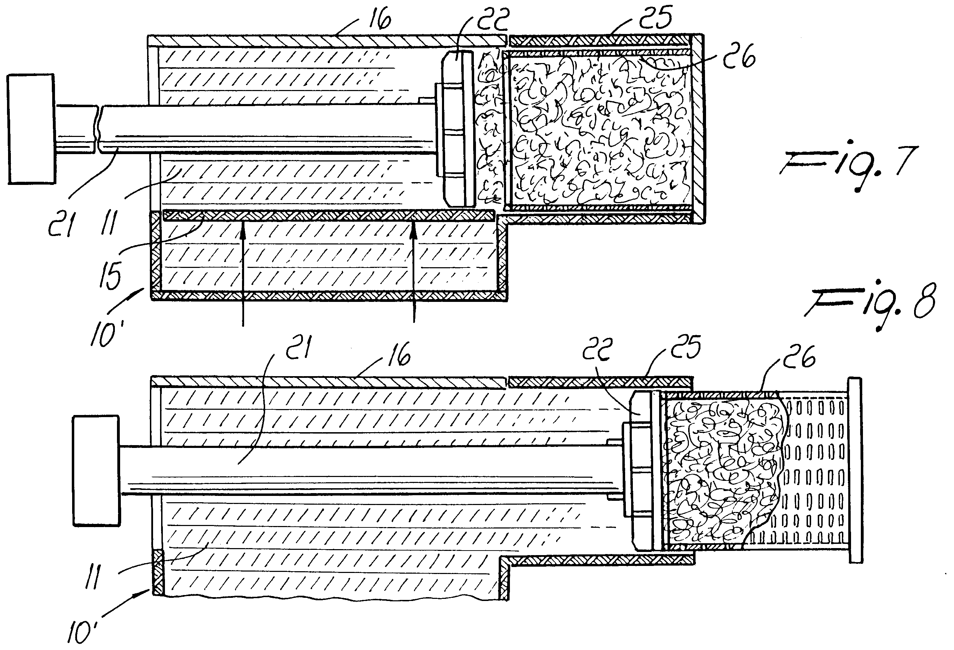

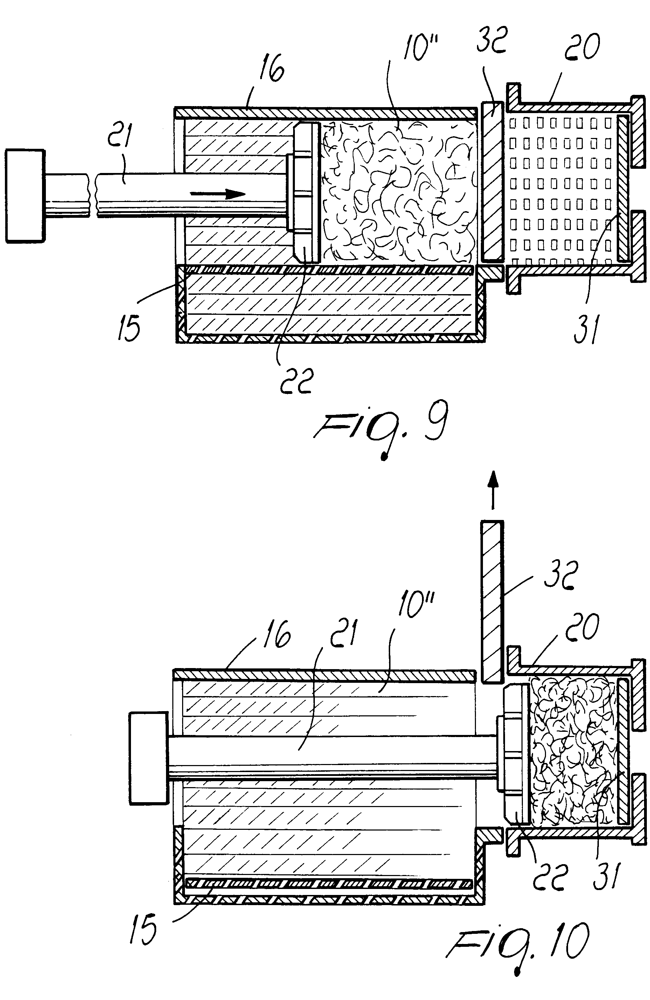

During pressing, the contrast piston 30 is arranged, through the pasteurization container, at the end of the compaction chamber 10; once the intended density of the compacted waste has been reached, the compacted block is transferred, as shown in figure 5, and is introduced in the container 20, which is closed by the cover 22 and by the panel 31.

According to an alternative embodiment, the container 20 can have, instead of the closure panel 31, a bottom panel of its own which, during the pressing step, is arranged so as to close the chamber 10 and then, during the transfer of the compacted block, is arranged so as to close the container 20, on the opposite side with respect to the cover 22.

The steps for volume reduction, final draining, high-pressure compaction, and transfer into the pasteurization container all occur in the same station with a substantially continuous cycle.

Once the pressing cycle has ended, the compacted block is placed inside the container 20; since said container does not have to withstand the pressing pressures, it is thinner than the walls of the chamber; the container is then removed and sent to the stabilization ovens 40, where thermal pasteurization of the compacted material is performed.

In these conditions, the material has a low heat transmission coefficient and pasteurization has the purpose of eliminating the residual moisture and most of all of stabilizing the organic substances, so as to make them stable and thus unaffected by putrefaction.

The pasteurization oven 40 being used is preferably of the forced-air chamber type, the air being produced by heat exchangers which are supplied in the primary circuit by catalytic methane-fired burners.

The operating temperature is approximately 250^{o}C for a time which is sufficient to inertize municipal solid waste and the like.

After the pasteurization step, the block is introduced in a cooling station 50 having a forced air system and heat recovery; the heat is sent both to the pasteurization oven and, as previously mentioned, to the homogenizing station in order to facilitate an initial evaporation of the liquids. The block can also be cooled, again with the forced-air system and with heat recovery, in the pasteurization station 40 without being moved again.

The container with the pasteurized material is sent to an extraction station 60 which allows to remove the compacted block with the aid of a piston; by means of a conveyor belt, said block is sent to storage or to the final destination of the compacted block, since the compacted block can be placed in a landfill or stored for other uses for which it may be intended.

The outward appearance is that of a highly compacted material with good mechanical characteristics, with a density of approximately 1.4 tons/m³, completely free from the typical odors of solid waste and with a heat value which can be estimated at approximately 3,500 kilocalories/kg.

According to a different embodiment, which is conceptually similar to the one described above, it is possible to provide for a compaction chamber, generally designated by the reference numeral 10' (figs. 7,8), having an extension 25 wherein an internal container 26 is provided which can be extracted once the material has been compacted.

In this embodiment, it is not necessary to provide for the counterthrust piston, since compaction occurs inside an internal container which again has a light structure, since the thrust is withstood by the outside walls of the chamber extension, the dimensions whereof are such as to withstand the pressures involved.

Refering to figures 9 and 10, a movable contrast panel 32 is provided, in the compaction chamber, indicated with 10'', at the opposite side with respect to the piston 21. In practice, the panel replaces the contrast piston 30 and is lowered during the pressing step and raised before the transfer of the compacted block in the pasteurization container 20.

In this case the bottom panel 31 is located in the container 20 before the pressing.

More than one block may be housed in the container 20, by correspondingly increasing the length of the container.

From the above description, it is thus evident that the invention achieves the intended aim and objects and in particular the fact is stressed that a method is provided which allows to reduce municipal solid waste and similar solid waste to an absolutely inert compacted block which is considerably smaller than its original dimensions and can be either freely stored in a landfill without provisions of any kind or can optionally be reused for various applications requiring inert materials.

The invention thus conceived is susceptible of numerous modifications and variations, all of which are within the scope of the inventive concept.

All the details may also be replaced with other technically equivalent elements.

In practice, the materials employed, as well as the contingent shapes and dimensions, may be any according to requirements.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for treating municipal solid waste and the like, characterized in that it consists in triturating the waste arriving from a storage area and draining the percolate; in homogenizing the triturated waste, with further percolate draining; in introducing the homogenized waste into a compaction and pressing chamber; in applying pressure to reduce the volume and bring the density to 1.4-1.5 tons/m³ with final percolate draining; in introducing the compacted block in a pasteurization container; in subjecting the container to thermal pasteurization without combustion to stabilize the organic materials and to destroy the microorganisms which produce fermentation; and in subjecting the pasteurized block to cooling with subsequent extraction from the container.

2. A method according to claim 1, characterized in that it consists in injecting hot air originated from said cooling of said container during the homogenization of the product.

3. A method according to the preceding claims, characterized in that it comprises a step for weighing the homogenized material to be introduced in said compaction chamber.

4. A method according to one or more of the preceding claims, characterized in that it consists in applying a logarithmically-increasing pressure in said compaction chamber.

5. A method according to one or more of the preceding claims, characterized in that the pressure reached in said compaction chamber is approximately 3,500 N/cm³.

6. A method according to one or more of the preceding claims, characterized in that said thermal pasteurization is performed at a temperature of approximately 250^{o}C.

7. A plant for treating municipal solid waste and the like, characterized in that it comprises a waste storage area (1), a stored waste triturator (2), a triturated waste homogenizer (4), a chamber (10,10',10'') for compacting the homogenized waste with means for compacting the waste to a density of 1.4-1.5 tons/m³, a container (20) for introducing the compacted waste which can be arranged at said compaction chamber (10), a thermal pasteurization oven (40), a cooling unit (50), and an extraction unit (60).

8. A plant according to claim 7, characterized in that said homogenizer (4) is connected to said cooling unit (50) to inject hot air for the evaporation of the contained liquids.

9. A plant according to one or more of claims 7-8, characterized in that it comprises a percolate storage tank (12) which is connected to said storage area (1), to said triturator (2), to said homogenizer (4), and to said compaction chamber (10).

10. A plant according to one or more of claims 7-9, characterized in that it comprises a unit (5) for weighing the homogenized material to be introduced in said compaction chamber (10).

11. A plant according to one or more of claims 7-10, characterized in that said compaction unit comprises a compacting and pressing chamber (10) arranged above a percolate collecting tank (12), said chamber (10) having lateral walls (11) with slots for the escape of the percolate, a bottom (15) being provided between said side walls (11) and being movable in an upward direction to provide a first volume reduction, a pasteurization container (20) being arrangeable next to said chamber (10) and having dimensions which correspond to the dimensions of a cover (22) which can be connected to a compaction piston (21) movable in said chamber (10), a contrast piston (30) being also provided which can be coupled to a closure panel (3) for closing said pasteurization container (20) in order to arrange said closure panel (31) at the end of said compaction chamber (10) during the pressing step, said compaction piston (21) and said contrast piston (30) being movable in order to transfer the compacted material into said pasteurization container (20).

12. A plant according to one or more of claims 7-11, characterized in that said cover (22) and said closure panel (31) have engagement means for coupling, respectively, to said compaction piston (21) and to said contrast piston (30), as well as automatic engagement systems for connection to said pasteurization container (20).

13. A plant according to one or more of claims 7-12, characterized in that it comprises a compaction chamber (10') having an extension (25) wherein an internal container (26) for accomodating the material during compaction is provided, said internal container (26) being removable from said compaction chamber (10').

14. A plant according to one or more of claims 7-13, characterized in that it comprises in said compaction chamber (10''), at the opposite side with respect to said compaction piston (21), a movable contrast panel (32) which can be removed after the pressing for the transfer of the compacted block in said container (20).
